(19) **Europäisches Patentamt**

**European Patent Office**

**Office européen des brevets**

(11) Veröffentlichungsnummer: **0 231 842 B1**

(12) **EUROPÄISCHE PATENTSCHRIFT**

(45) Veröffentlichungstag der Patentschrift: **17.06.92**

(51) Int. Cl.5: **C07C 227/04**, C07C 229/06, C07C 51/09, C07C 59/147

(21) Anmeldenummer: **87100992.4**

(22) Anmeldetag: **24.01.87**

(54) **Verfahren zur Herstellung von 6-Aminocapronsäure.**

(30) Priorität: **28.01.86 DE 3602374**

(43) Veröffentlichungstag der Anmeldung:
**12.08.87 Patentblatt 87/33**

(45) Bekanntmachung des Hinweises auf die
Patenterteilung:
**17.06.92 Patentblatt 92/25**

(84) Benannte Vertragsstaaten:
**BE CH DE FR GB IT LI NL**

(56) Entgegenhaltungen:
DE-A- 952 442
GB-A- 2 046 253
US-A- 2 777 873

(73) Patentinhaber: **BASF Aktiengesellschaft
Carl-Bosch-Strasse 38
W-6700 Ludwigshafen(DE)**

(72) Erfinder: **Vagt, Uwe, Dr.
Paul-Neumann-Strasse 44
W-6720 Speyer(DE)**
Erfinder: **Fischer, Rolf, Dr.
Bergstrasse 98
W-6900 Heidelberg(DE)**
Erfinder: **Merger, Franz, Dr.
Max-Slevogt-Strasse 25
W-6710 Frankenthal(DE)**
Erfinder: **Hutmacher, Hans-Martin, Dr.
Ruedigerstrasse 70
W-6700 Ludwigshafen(DE)**

**Beschreibung**

Gegenstand der Erfindung ist ein Verfahren zur Herstellung von 6-Aminocapronsäure aus 5-Formylvaleriansäureestern.

Nach einem in J. Am. Chem. Soc., Band 64 (1942), Seite 1416 ff beschriebenen Verfahren erhält man 5-Formylvaleriansäure durch Umsetzen von 2-Hydroxicyclohexanon mit Bleitetraacetat in Eisessig. Dieses Verfahren hat den Nachteil, daß es aufwendige Ausgangsstoffe benötigt und nur in ungenügenden Ausbeuten verläuft. Zudem ist es durch den Zwangsanfall von Bleiverbindungen, die entsorgt werden müssen, belastet. Nach einem anderen in Chem. Berichte, Band 72 (1939), Seiten 1194 ff beschriebenen Verfahren erhält man 5-Formylvaleriansäure durch Umsetzen von Cyclohexanon mit alkalischem Wasserstoffperoxid. Hierbei erzielt man jedoch nur geringe Selektivitäten und benötigt lange Reaktionszeiten. Nach einem weiteren in der EP-Anmeldung 91 091 beschriebenen Verfahren stellt man 5-Formylvaleriansäure durch Oxidation von Cyclohexanon mit molekularem Sauerstoff in Gegenwart von homogenen Katalysatoren wie Eisen(III)chlorid und Thioharnstoff her. Nach diesem Verfahren erhält man jedoch ausreichende Selektivitäten nur bei Umsätzen von weniger als 50 %. Darüber hinaus müssen die homogen gelösten Katalysatoren aus dem Reaktionsgemisch abgetrennt werden, was aufwendig ist. In der US-A 2,777,873 wird die Herstellung von 6-Aminocapronsäureestern durch Umsetzen von 5-Formylvaleriansäureestern mit Ammoniak und Wasserstoff in Gegenwart von Katalysatoren beschrieben.

Aus der japanischen Patentveröffentlichung 21 138/66 ist auch schon ein verfahren bekannt, bei dem man $\delta$-Formylvaleriansäure, Ammoniak und Wasser als Verdünnungsmittel unter Mitverwendung von Wasserstoff und Hydrierkatalysatoren bei Temperaturen über 100°C umsetzt. Hierbei erhält man jedoch Caprolactam.

Es war deshalb die technische Aufgabe gestellt, ein Verfahren zur Verfügung zu stellen, bei dem man aus 5-Formylvaleriansäureestern 6-Aminocapronsäure mit hohen Ausbeuten und kurzen Reaktionszeiten mit leicht abtrennbaren Katalysatoren und unter Bildung von wenig Nebenprodukten erhält.

Diese Aufgabe wird gelöst in einem Verfahren zur Herstellung von 6-Aminocapronsäure, dadurch gekennzeichnet, daß man

a) 5-Formylvaleriansäureester mit Wasser in Gegenwart von nicht oxidierenden Mineralsäuren, Lewissäuren, niederen Fettsäuren, Sulfonsäuren oder stark sauren Ionenaustauschern bei einer Temperatur von 30 bis 200°C unter fortlaufendem Abtrennen der anfallenden Alkohole durch Destillation aus dem Reaktionsgemisch umsetzt und

b) die so erhaltene 5-Formylvaleriansäure in Form einer wässrigen Lösung, wie sie in Stufe a) anfällt, mit überschüssigem Ammoniak und Wasserstoff in Gegenwart von Hydrierkatalysatoren und unter Reaktionsbedingungen inerten Lösungsmitteln bei einer Temperatur von 50 bis 150°C unter erhöhtem Druck umsetzt.

Das neue Verfahren hat den Vorteil, daß es auf einfache Weise gelingt, aus 5-Formylvaleriansäureestern 6-Aminocapronsäure in hohen Ausbeuten herzustellen. Darüber hinaus hat das neue Verfahren den Vorteil, daß es in kurzer Zeit verläuft und wenige Nebenprodukte anfallen. Ferner hat das neue Verfahren den Vorteil, daß leicht abtrennbare Katalysatoren Verwendung finden. Das neue Verfahren ist insofern bemerkenswert, als zu erwarten war, daß 5-Formylvaleriansäure in saurem Medium inter- oder intramolekulare Aldolkondensationen (Bildung von fünfgliedrigen Ringen) eingehen würde. So ist aus Houben-Weyl, Methoden der org. Chemie, Band VII/1, Seite 87 bekannt, daß Adipindialdehyd in saurem Medium eine intramolekulare Aldolkondensation eingeht.

Bevorzugte 5-Formylvaleriansäureester, die in Stufe a eingesetzt werden, leiten sich von Alkanolen mit 1 bis 12 Kohlenstoffatomen, Cycloalkanolen mit 5 bis 7 Kohlenstoffatomen, Aralkanolen mit 7 bis 8 Kohlenstoffatomen oder Phenolen mit 6 bis 8 Kohlenstoffatomen ab. Besonders bevorzugte Ausgangsstoffe sind 5-Formylvaleriansäure-$C_1$- bis -$C_4$-Alkylester. Geeignete Ausgangsstoffe sind beispielsweise 5-Formylvaleriansäuremethylester, 5-Formylvaleriansäureethylester, 5-Formylvaleriansäure-n-propylester, 5-Formylvaleriansäuren-i-propylester, 5-Formylvaleriansäure-n-butylester, 5-Formylvaleriansäure-2-ethylhexylester, 5-Formylvaleriansäurecyclohexylester oder 5-Formylvaleriansäurephenylester. Besondere technische Bedeutung hat 5-Formylvaleriansäuremethylester erlangt. 5-Formylvaleriansäureester sind nach dem in der ER-Anmeldung 31 000 beschriebenen Verfahren durch Hydroformylierung von 4-Pentensäureestern leicht zugänglich.

Im allgemeinen wendet man je Mol 5-Formylvaleriansäureester 1 bis 200 Mol, insbesondere 50 bis 150 Mol Wasser an. Darüber hinaus können unter Reaktionsbedingungen inerte Lösungsmittel mitverwendet werden. Geeignete Lösungsmittel sind beispielsweise Kohlenwasserstoffe wie Cyclohexan oder Toluol, Halogenkohlenwasserstoffe wie Dichlormethan, Tetrachlormethan, Ether wie Dioxan oder Diglyme. Falls Lösungsmittel mitverwendet werden, setzt man 5-Formylvaleriansäureester als 1 bis 90 gew.%ige Lösung, insbesondere 5 bis 20 gew.%ige Lösung ein.

Die Umsetzung wird bei einer Temperatur von 30 bis 200°C durchgeführt. Vorteilhaft wendet man eine Temperatur von 50 bis 120°C an. Im allgemeinen führt man die Umsetzung unter Atmosphärendruck durch. Es ist jedoch auch möglich, schwach verminderten Druck oder schwach erhöhten Druck, z.B. bis zu 20 bar anzuwenden.

Die Umsetzung wird unter Mitverwendung von Sulfonsäuren, Lewissäuren, nicht oxydierenden Mineralsäuren, niederen Fettsäuren oder stark sauren Kationenaustauschern durchgeführt. Geeignete saure Mittel sind beispielsweise nicht-oxidierende Mineralsäuren wie Schwefelsäure, Chlorwasserstoffsäure, Bromwasserstoffsäure, Sulfonsäuren, wie p-Toluolsulfosäure oder Lewissäuren wie Bortrifluorid oder Zinkchlorid, ferner niedere aliphatische Carbonsäuren wie Ameisensäure, Essigsäure oder Propionsäure sowie stark saure Kationenaustauscher. Solche stark sauren Kationenaustauscher sind beispielsweise aufgebaut aus vernetztem Polystyrol mit Sulfonsäuregruppen, Phenolharzen mit Sulfonsäuregruppen oder stellen saure Zeolithe dar.

Vorteilhaft wendet man Sulfonsäuren, Lewissäuren oder nicht-oxidierende Mineralsäuren in katalytischen Mengen, z.B. von 0,002 bis 0,25 Mol je Mol 5-Formylvaleriansäureester an. Aliphatische Carbonsäuren werden im allgemeinen in Mengen von 0,1 bis 1 Mol je Mol 5-Formylvaleriansäureester eingesetzt.

Besonders bevorzugt werden stark saure Kationenaustauscher angewandt.

Das Verfahren kann chargenweise oder vorteilhaft kontinuierlich, z.B. in einer Rührkesselkaskade durchgeführt werden. Hierbei wird der bei der Hydrolyse anfallende Alkohol fortlaufend durch Destillation aus dem Reaktionsgemisch abzutrennt. Bei der Verwendung von stark sauren Kationenaustauschern ist es vorteilhaft, die Umsetzung so durchzuführen, daß man die stark sauren Kationenaustauscher, z.B. in einem Rohrreaktor fest anordnet und das Reaktionsgemisch in Rieselfahrweise darüber leitet. In einer besonders vorteilhaften Ausführungsform wird das Reaktionsgemisch zunächst in Rieselfahrweise durch eine erste Reaktionszone mit fest angeordneten stark sauren Kationenaustauschern geleitet und dann in einer zweiten Reaktionszone über fest angeordnete stark saure Kationenaustauscher im Kreis geführt und das Reaktionsgemisch in dem Maße entnommen, wie es der ersten Zone zugeführt wird. Vorteilhaft wird aus dem so erhaltenen Umsetzungsgemisch nicht umgesetzter 5-Formylvaleriansäureester z.B. mit Kohlenwasserstoffen wie Cyclohexan extrahiert und zweckmäßig wieder verwendet.

Nach einer anderen vorteilhaften Arbeitsweise leitet man 5-Formylvaleriansäureester und Wasser im Überschuß durch eine mit stark sauren Kationenaustauschern beschichtete Kolonne, destilliert am oberen Ende Alkohole ab und entnimmt am unteren Ende eine wäßrige Lösung von 5-Formylvaleriansäure.

Man verwendet die wäßrige Lösung der 5-Formylvaleriansäure, die beispielsweise einen Gehalt von 3 bis 15 Gew.% hat, direkt für die Herstellung von 6-Aminocapronsäure.

In einer zweiten Stufe (Stufe b) wird dann die so erhaltene 5-Formylvaleriansäure mit überschüssigem Ammoniak und Wasserstoff in Gegenwart von Hydrierkatalysatoren und Lösungsmitteln bei einer Temperatur von 50 bis 150°C unter erhöhtem Druck umgesetzt.

5-Formylvaleriansäure wird im allgemeinen als 1 bis 50 gew.%ige, vorzugsweise 2 bis 35 gew.%ige, insbesondere 5 bis 25 gew.%ige Lösung verwendet. Die Lösungen enthalten überwiegend Monomere und wenig niedermolekulare oligomere 5-Formylvaleriansäure. Geeignete Lösungsmittel sind beispielsweise Wasser, ein- oder mehrwertige Alkohole mit 1 bis 5 Kohlenstoffatomen, Ether wie Dioxan, Tetrahydrofuran, Diglyme oder Gemische der genannten Lösungsmittel.

Vorzugsweise wird Wasser als Lösungsmittel verwendet. Insbesondere hat es sich bewährt, wenn man die wäßrige Lösung von 5-Formylvaleriansäure, wie sie in der Stufe a anfällt, verwendet.

In der Regel wendet man je Mol 5-Formylvaleriansäure 2 bis 50 Mol, insbesondere 4 bis 30 Mol Ammoniak an.

Die Umsetzung führt man bei einer Temperatur von 50 bis 150°C, vorteilhaft 70 bis 130°C durch. Im allgemeinen wendet man einen Druck von 10 bis 400 bar, vorteilhaft von 20 bis 300 bar, insbesondere 50 bis 250 bar an. Wasserstoff wird in der Regel in Mengenverhältnissen von 1 bis 10 Mol je Mol 5-Formylvaleriansäure eingesetzt. Vorzugsweise verwendet man Wasserstoff im Überschuß, z.B. 2 bis 5 Mol je Mol 5-Formylvaleriansäure.

Geeignete Hydrierkatalysatoren sind Metalle der 8. Gruppe des periodischen Systems, wie Nickelkatalysatoren, Cobaltkatalysatoren oder Edelmetallkatalysatoren, wie Ruthenium, Platin oder Palladium. Die Katalysatoren können aktivierende Zusätze wie Zirkon, Mangan, Kupfer oder Chrom enthalten. Die Katalysatoren können als Vollkatalysatoren oder auf Trägern wie Aluminiumoxid, Kieselgel, Tonerde oder Aktivkohle niedergeschlagen verwendet werden. Geeignet sind auch Skelettkatalysatoren.

Geeignete Katalysatoren enthalten z.B. 80 bis 100 Gew.% Kobalt und/oder Nickel, bezogen auf den Metallgehalt des Katalysators. Neben Kobalt und/oder Nickel können die Katalysatoren Metalle wie Kupfer oder Chrom, z.B. bis zu 20 Gew.% bezogen auf den Metallgehalt enthalten. Die Katalysatoren können auf Träger, z.B. auf Kieselsäuresilikate, Aluminiumphosphat, Borphosphat, Aluminiumoxid oder Aktivkohle aufgebracht sein. Der Metallgehalt der Trägerkatalysatoren beträgt zweckmäßig 5 bis 80 Gew.%, bezogen auf die gesamte Katalysatormasse.

Andere geeignete Katalysatoren sind solche, die man durch Calcinieren von Verbindungen der Formel I $[(Mg_aNi(II)_bCo(II)_c)Al_2]CO_3(OH)_{16}X4H_2O$, in der a für ganze oder Dezimalzahlen von 0 bis 4 steht und b und c für ganze Zahlen oder Dezimalzahlen von 0 bis 6 stehen, mit der Maßgabe, daß $2 \times (a + b + c) = 12$ ist bei Temperaturen von 200 bis 600°C und anschließend Reduktion mit Wasserstoff bei erhöhter Temperatur hergestellt worden. Bevorzugt geht man von folgenden der Formel I entsprechenden Verbindungen aus:

$Ni_6Al_2(OH)_{16}CO_3 \cdot 4 H_2O$,
$Ni_5MgAl_2(OH)_{16}CO_3 \cdot 4 H_2O$,
$Co_6Al2(OH)_{16}CO_3 \cdot 4 H_2O$,
$Co_5MgAl_2(OH)_{16}CO_3 \cdot 4 \cdot H_2O$.

Die Verbindungen der Formel I erhält man beispielsweise wie folgt: Nickel, Aluminium, Kobalt und Magnesium werden in Form ihrer wasserlöslichen Salze wie Chloride, Sulfate oder vorzugsweise Nitrate gemeinsam in Wasser gelöst und zwar in einem Mengenverhältnis, das der gewünschten Zusammensetzung des Katalysators möglichst nahekommt und in seiner Stöchiometrie der Formel I gehorcht.

Die Metallsalzlösung soll insgesamt etwa 0,5 bis 3 molar, vorzugsweise 1,0 bis 2 molar an Metallionen sein. Sie wird auf Temperaturen von 50 bis 100°C, vorzugsweise 80 bis 100°C erhitzt und innerhalb von 0,5 bis 10, vorzugsweise 1 bis 3 Minuten mit einer äuqivalenten Menge oder bevorzugt einem geringen Überschuß, einer auf 50 bis 100°C, vorzugsweise 80 bis 100°C erhitzten 1 bis 3, vorzugsweise 1,5 bis 2,5 molaren Lösung eines Alkalibicarbonats vereinigt. Vorteilhaft arbeitet man mit einem Überschuß an Alkalibicarbonat, der bis zu 20 Gew.%, vorzugsweise 0,5 bis 3 Gew.%, bezogen auf die theoretische Menge des Bicarbonats beträgt. Nach der Zugabe der Metallsalzlösung, nach Vereinigung der beiden Lösungen wird zweckmäßig noch 10 bis 30, vorzugsweise 15 bis 20 Minuten gerührt und dann der entstandene Niederschlag abfiltriert, mit Wasser gewaschen und bei einer Temperatur von 50 bis 200°C, vorzugsweise 100 bis 160°C getrocknet. Die basischen Carbonate werden in nahezu quantitativen Ausbeuten erhalten. Als Alkalibicarbonate kommen insbesondere Natriumbicarbonat oder Kaliumbicarbonat in Betracht. Es ist aber auch möglich, Ammoniumbicarbonat bei der Fällung zu verwenden. Desgleichen können auch Mischungen der genannten Bicarbonate eingesetzt werden. Außerdem ist es möglich die Fällung der Metallionen mit Lösungen von Alkalicarbonaten in Natriumcarbonat und/oder Kaliumcarbonat vorzunehmen, wenn man während des Fällens Kohlendioxid in die vorgelegte Alkalicarbonatlösung einleitet, was im Endeffekt auch auf eine Fällung mit Bicarbonat hinausläuft.

Vorteilhaft wendet man beim Calcinieren Temperaturen von 250 bis 400°C, z.B. für eine Dauer von 5 bis 40, insbesondere 15 bis bis 30 Stunden an. Vor der eigentlichen Verwendung der Katalysators wird dieser mit Wasserstoff vorteilhaft bei einer Temperatur von 180 bis 500°C, vorzugsweise 250 bis 450°C, z.B. innerhalb von 5 bis 100 Stunden, vorteilhaft 10 bis 25 Stunden reduziert.

Bei der Hydrierung hält man vorteilhaft eine Verweilzeit von 1 bis 120 Minuten, insbesondere 10 bis 60 Minuten ein. Ferner hat sich eine Belastung von 0,2 bis 2 kg 5-Formyl-Valeriansäure je Liter Katalysator und Stunde bewährt.

Die Umsetzung kann diskontinuierlich, z.B. in einem Druckgefäß oder kontinuierlich in einer Reaktionszone mit fest angeordneten Katalysatoren, Sumpf- oder Rieselfahrweise durchgeführt werden. Es hat sich besonders bewährt, wenn eine Rückvermischung während der Umsetzung vermieden wird.

Aus dem erhaltenen Reaktionsgemisch wird 6-Aminocapronsäure, das als inneres Salz anfällt, in üblicher Weise z.B. durch Kristallisation abgetrennt.

Die nach dem Verfahren der Erfindung hergestellte 6-Aminocapronsäure eignet sich zur Herstellung von Caprolactam oder direkt zur Herstellung von Polyamiden.

Das Verfahren nach der Erfindung sei an folgenden Beispielen veranschaulicht.

Beispiel 1

36 g 5-Formylvaleriansäuremethylester, 90 g Wasser und 0,5 g Schwefelsäure wurden 3,5 Stunden unter Rückfluß erhitzt, wobei gebildetes Methanol ständig abdestilliert wurde. Nach fraktionierender Vakuumdestillation wurden 18,5 g 5-Formylvaleriansäure erhalten (Ausbeute 57 % d.Th.).

Sdp. 106 bis 112°C/l mbar; 13 C-NMR-Spektrum in D-Chloroform: Chemische Verschiebungen, relativ zu Tetramethylsilan als innerem Standard = 21, 24, 34 und 44 ppm (4-Methylengruppen), 178 ppm (Formylgruppe) und 203 ppm (Carboxylgruppe).

Beispiel 2

36 g 5-Formylvaleriansäuremethylester, 90 g Wasser und 1g p-Toluolsulfonsäure wurden 8 Stunden unter Rückfluß erhitzt, wobei gebildetes Methanol ständig abdestilliert wurde. Nach fraktionierender Vakuumdestillation wurden 13,3 g 5-Formylvaleriansäure erhalten (Ausbeute 41 % d.Th.).

Beispiel 3

36 g 5-Formylvaleriansäuremethylester, 90 g Wasser und 2 g Kationenaustauscher aus vernetztem Polystyrol mit Sulfonsäuregruppen wurden 8,5 Stunden unter Rückfluß erhitzt, wobei gebildetes Methanol ständig abdestilliert wurde. Nach fraktionierender Vakuumdestillation wurden 24 g 5-Formylvaleriansäure erhalten (Ausbeute 74 % d.Th.).

Beispiel 4

5-Formylvaleriansäuremethylester (5-FVSE; 5-10 ml/h) und Wasser (50 bis 100 ml/h wurden kontinuierlich über zwei beheizbare Doppelmantelstahlrohrreaktoren (Innentemperatur 60 bis 100°C) gepumpt, die mit einem Kationenaustauscher aus vernetztem Polystyrol mit Sulfonsäuregruppen gefüllt waren (Reaktor I: 100 ml Ionenaustauscher, ohne Kreislauf; Reaktor II: 50 ml Ionenaustauscher, 0 bis 30 l/h, Kreislauf).
In den Austrägen wurden folgende Mengen 5-Formylvaleriansäure (5-FVS) und 5-FVSE gaschromatographisch bestimmt:

| No. | Temp. | Kreisl.R II [l/h] | 5-FVS [Gew.%] | 5-FVSE [Gew.%] | Ausbeute [d. Th.] | Selekt. [%] |
|---|---|---|---|---|---|---|
| 41 | 60°C | 15 | 10,39 | 1,89 | 78 | 89 |
| 42 | 80°C | 15 | 6,36 | 0,59 | 78 | 84 |
| 43 | 100°C | 15 | 4,00 | 0,38 | 53 | 56 |

Ein Teil des Reaktionsaustrages (Reaktionstemperatur 60°C) wurde im Vakuum fraktionierend destilliert.
Hierbei wurden 75 % d.Th. 5-FVS vom Sdp. 102 bis 123°C/1 mbar erhalten.
Die Reaktionsausträge wurden mit Cyclohexan extrahiert.
In den wäßrigen Phasen wurden nach der Extraktion folgende Mengen 5-FVS und 5-FVSE gaschromatographisch bestimmt:

| No. | 5-FVS [Gew.%] | 5-FVSE [Gew.%] |
|---|---|---|
| 41 | 10,28 | 0,21 |
| 42 | 6,23 | 0,16 |
| 43 | 3,97 | <0,01 |

Beispiel 5

a) 5-Formylvalerinsäure durch Verseifung von 5-Formylvaleriansäuremethylester

5-Formylvaleriansäuremethylester (5-FVSE; 5 ml/h) und Wasser (50 ml/h) wurden kontinuierlich über zwei beheizbare Doppelmantelstahlrohrreaktoren (Innentemperatur 60°C) gepumpt, die mit einem stark sauren Ionenaustauscher gefüllt waren. (Reaktor I: ohne Kreislauf: Reaktor II: 15 l/h Kreislauf).
Im Austrag wurden folgende Mengen 5-Formylvaleriansäure (5-FVS) und 5-FVSE gaschromatographisch bestimmt:

| 5-FVS | 5-FVSE | Ausbeute | Selektivität |
|---|---|---|---|
| [Gew.%] | [Gew.%] | [% d.Th.] | [%] |
| 7,03 | 0,80 | 78 | 85 |

Der Reaktionsaustrag wurde mit Cyclohexan extrahiert. In der wäßrigen Phase wurden nach der Extraktion folgende Mengen 5-FVS und 5-FVSE gaschromatographisch bestimmt:

| 5-FVS [Gew.%] | 5-FVSE [Gew.%] |
|---|---|
| 6,23 | 0,04 |

b) 6-Aminocapronsäure durch aminierende Hydrierung von 5-Formylvaleriansäure

100 ml 5-Formylvaleriansäurelösung in Wasser (6,23 Gew.% 5-FVS; Reaktionsaustrag aus a) nach der Extraktion unumgesetzten Esters mit Cyclohexan) wurden bei 110°C/150 bar Wasserstoff zu einer Suspension von 10 g Ru-Zr-Katalysator (0,5 Gew.% Ru und 1 Gew.% Zr auf Aluminiumoxid) in 60 g 12,5 %igem wäßrigen Ammoniak in einem 300 ml Stahlrührautoklaven gepumpt und anschließend 2 Stunden bei 110°C gerührt.

Die Bestimmung der Aminocapronsäure im Reaktionsaustrag erfolgte durch quantitative HPLC-Chromatographie und ergab eine Ausbeute von 77 % d.Th.

Beispiel 6

100 ml einer 14,8 gew.%igen 5-Formylvaleriansäurelösung in Wasser wurden bei 70 bis 130°C/150 bar Wasserstoff zu einer Suspension des Katalysators in wäßrig, ammoniakalischer Lösung in einem 300 ml Stahlrührautoklaven gepumpt und anschließend 2 Stunden bei Temperatur gerührt.

Die Bestimmung der Aminocapronsäure im Reaktionsaustrag erfolgte durch quantitative HPLC-Chromatographie.

| No. | Temp. [°C] | Katalysator | Ausbeute 6-ACS [% d.Th.] |
|---|---|---|---|
| 6.1 | 70 | 2 g RANEY-Nickel | 57 |
| 6.2 | 110 | 2 g RANEY-Cobalt | 57 |
| 6.3 | 130 | 2 g RANEY-Nickel | 57 |

Vergleichsbeispiel

10 g trimere 5-Formylvaleriansäure, 60 g 12,5 %iges wäßriges Ammonik und 2 g RANEY-Nickel wurden 5 Stunden bei 110°C/150 bar Wasserstoff in einem 300 ml Stahlrührautoklaven gerührt.

Im Reaktionsaustrag wurden durch quantitative HPLC-Chromatographie 31 % d.Th. 6-Aminocapronsäure bestimmt.

**Patentansprüche**

1. Verfahren zur Herstellung von 6-Aminocapronsäure, dadurch gekennzeichnet, daß man
   a) 5-Formylvaleriansäureester mit Wasser in Gegenwart von nicht oxidierenden Mineralsäuren, Lewissäuren, niederen Fettsäuren, Sulfonsäuren oder stark sauren Ionenaustauschern bei einer Temperatur von 30 bis 200°C unter fortlaufendem Abtrennen der anfallenden Alkohole durch Destillation aus dem Reaktionsgemisch umsetzt und
   b) die so erhaltene 5-Formylvaleriansäure in Form einer wässrigen Lösung, wie sie in Stufe a) anfällt, mit überschüssigem Ammoniak und Wasserstoff in Gegenwart von Nickel, Kobalt oder Edelmetallkatalysatoren der 8. Gruppe und unter Reaktionsbedingungen inerten Lösungsmitteln bei einer Temperatur von 50 bis 150°C unter erhöhtem Druck umsetzt.

2. Verfahren nach Anspruch 1, dadurch gekennzeichnet, daß man 50 bis 150 Mol Wasser je Mol 5-Formylvaleriansäureester anwendet.

3. Verfahren nach den Ansprüchen 1 und 2, dadurch gekennzeichnet, daß man 5-Formylvaleriansäureester mit Wasser über 2 hintereinandergeschaltete Reaktionszonen, die mit stark sauren Kationenaustauschern beschickt sind, leitet, wobei in der zweiten Reaktionszone das Reaktionsgemisch im Kreis geführt wird und die entnommene wäßrige Lösung mit Kohlenwasserstoffen extrahiert wird.

4. Verfahren nach den Ansprüchen 1 bis 3, dadurch gekennzeichnet, daß man je Mol 5-Formylvaleriansäure 4 bis 30 Mol Ammoniak verwendet.

## Claims

1. A process for preparing 6-aminocaproic acid, which comprises
   a) reacting a 5-formylvaleric ester with water in the presence of a non-oxidizing mineral acid, a Lewis acid, a lower fatty acid, a sulfonic acid or a strongly acidic ion exchanger at 30-200°C with continuous removal of the resulting alcohol from the reaction mixture by distillation and
   b) reacting the 5-formylvaleric acid thus obtained in the form of an aqueous solution as obtained in stage a) with excess ammonia and hydrogen in the presence of nickel, cobalt or a noble metal catalyst of group 8 and a solvent which is inert under the reaction conditions at 50-150°C under superatmospheric pressure.

2. A process as claimed in claim 1, wherein 50-150 moles of water are used per mole of 5-formylvaleric ester.

3. A process as claimed in claim 1 or 2, wherein the 5-formylvaleric ester is passed with water through two reaction zones connected in series which are packed with a strongly acid cation exchanger, in the second reaction zone the reaction mixture being recirculated and the aqueous solution removed being extracted with a hydrocarbon.

4. A process as claimed in any of claims 1 to 3, wherein from 4 to 30 moles of ammonia are used per mole of 5-formylvaleric acid.

## Revendications

1. Procédé de préparatian d'acide 6-aminocaproïque, caractérisé en ce que
   a) on fait réagir un ester d'acide 5-formylvalérique avec de l'eau en présence d'acides minéraux non oxydants, d'acides de Lewis, d'acides gras inférieurs, d'acides sulfoniques au d'échangeurs d'tons fortement acides, à une température de 30 à 200°C et en séparant constamment du mélange réactionnel par distillation les alcools qui se forment, et
   b) on fait réagir l'acide 5-formylvalérique ainsi obtenu sous forme d'une solution aqueuse, tel qu'il est formé dans l'étape a), avec de l'ammoniac et de l'hydrogène en excès, en présence de catalyseurs de nickel, de cobalt au de métaux précieux du groupe VIII et dans les conditions de réaction suivantes: solvant inerte, température de 50 à 150°C et pression élevée.

2. Procédé selon la revendicatian 1, caractérisé en ce qu'on utilise de 50 à 150 moles d'eau par mole d'ester d'acide 5-formylvalérique.

3. Procédé selon la revendicatian 1 ou 2, caractérisé en ce qu'on fait passer l'ester d'acide 5-formylvalérique avec l'eau à travers deux zones de réaction qui sont disposées à la suite l'une de l'autre et qui sont garnies d'échangeurs de cations fortement acides, le mélange réactiannel étant mis en circulation dans la seconde zone de réaction et la solution aqueuse prélevée étant extraite avec des hydrocarbures.

4. Procédé selon l'une quelconque des revendications 1 à 3, caractérisé en ce qu'on utilise, par mole d'acide 5-formvlvalérique, de 4 à 30 moles d'ammoniac.